# EUROPÄISCHE PATENTSCHRIFT

(11) **EP 1 388 007 B1**
(45) Veröffentlichungstag und Bekanntmachung des Hinweises auf die Patenterteilung: **19.09.2007**
(21) Anmeldenummer: 02745052.7
(22) Anmeldetag: 15.05.2002
(51) Int. Cl.: G01N 33/483, G01N 27/62, G01N 33/68

(54) **VERFAHREN ZUR QUALITATIVEN UND/ODER QUANTITATIVEN BESTIMMUNG VON GATTUNG, ART, RASSE UND/ODER GEOGRAPHISCHER HERKUNFT BIOLOGISCHER MATERIALIEN**
METHOD FOR QUALITATIVE AND/OR QUANTITATIVE DETERMINATION OF GENDER, SPECIES, RACE AND/OR GEOGRAPHICAL ORIGIN OF BIOLOGICAL MATERIALS
PROCEDE DE DETERMINATION QUALITATIVE ET/OU QUANTITATIVE DU GENRE, DE L'ESPECE, DE LA RACE ET/OU DE L'ORIGINE GEOGRAPHIQUE DE MATERIAUX BIOLOGIQUES

(30) Priorität: 15.05.2001 DE 10123711
(43) Veröffentlichungstag der Anmeldung: 11.02.2004
(73) Patentinhaber: Altmeyer, Wolfgang, 66763 Dillingen (DE)
(72) Erfinder: ALTMEYER, Wolfgang, 66763 Dillingen (DE); HOLLEMEYER, Klaus, 66540 Neunkirchen (DE); HEINZLE, Elmar, 66123 Saarbrücken (DE); EWEN, Heiko, 66701 Beckingen (DE)
(74) Vertreter: Vièl, Christof
(86) Internationale Anmeldenummer: PCT/DE2002/001737
(87) Internationale Veröffentlichungsnummer: WO 2002/093166

(56) Entgegenhaltungen:
- WO-A-96/36986
- WO-A-98/07036
- FR-A- 2 647 906
- US-A- 5 952 653
- US-A- 5 969 350
- US-B1- 6 177 266
- DATABASE WPI Section Ch, Week 198646 Derwent Publications Ltd., London, GB; Class B04, AN 1986-304098 XP002214742 & SU 1 222 246 A (BELO FORENSIC MED INST), 30. März 1986 (1986-03-30)
- KRISHNAMURTHY T ET AL: "Detection of pathogenic and non-pathogenic bacteria by matrix-assisted laser desorption/ionization time-of-flight mass spectrometry" RAPID COMMUNICATIONS IN MASS SPECTROMETRY, Bd. 10, Nr. 8, Juni 1996 (1996-06), Seiten 883-888, XP009013355 ISSN 0951-4198

## Beschreibung

Die vorliegende Erfindung betrifft ein Verfahren zur qualitativen und/oder quantitativen Bestimmung von Gattung, Art, Rasse und/oder geographischer Herkunft biologischer Materialien auf der Grundlage von Schuppen, Haaren, Federn, Daunen und/oder Horn sowie die Verwendung des Verfahrens.

Zur Bestimmung von Gattung, Art, Rasse und/oder geographischer Herkunft in biologischen Proben wurden bisher unterschiedliche Methoden angewandt. Zu nennen sind makroskopisch und mikroskopisch-visuelle Untersuchungen, wobei eine sichere Zuordnung aufgrund der vielfältigen Übergangsformen der untersuchten Merkmale in biologischen Proben oft schwierig ist und ein hohes Maß an Erfahrung erfordert ("Determination of Feather and Down Species", Proposed IDFB Method-1 May 1999, IDFB Handbook, Kopie s. Anlage). Insbesondere versagt diese Methode bei Proben, in denen die zu untersuchenden Merkmale nicht angesprochen werden können.

Eine weitere Unterscheidungsmöglichkeit ergibt sich aus der Anwendung proteinchemischer Methoden, wobei Artbestimmungen bisher durch die elektrophoretische Auftrennung von Gesamtproteinproben unter denaturierenden und nichtdenaturierenden Bedingungen ("Nachweis der Tierart bei nativem Muskelfleisch in Polyacrylamid-Gelen mit Hilfe der Standard-Elektrophorese (PAGE), Amtliche Sammlung von Untersuchungsverfahren nach § 35 LMBG, Methode 06.00-27, Dezember 1988, Herausgeber und Redaktion: bgvv, Bundesinstitut für gesundheitlichen Verbraucherschutz und Veterinärmedizin, Band I/3, Lebensmittel (L), Teil 2, Beuth Verlag GmbH, Berlin, Köln, Wien, Zürich) sowie durch isoelektrische Fokussierung ("Nachweis der Tierart bei Milch, Milchprodukten und Käse mit Hilfe der isoelektrischen Fokussierung (PAGIF)", Amtliche Sammlung von Untersuchungsverfahren nach § 35 LMBG, Methode 01.00-39, Januar 1995, Herausgeber und Redaktion: bgw, Bundesinstitut für gesundheitlichen Verbraucherschutz und Veterinärmedizin, Band 1/3, Lebensmittel (L), Teil 1 a, Beuth Verlag GmbH, Berlin, Köln, Wien, Zürich; 'Nachweis von Kuhmilchkasein in Käse aus Schaf-, Ziegen- oder Büffelmilch oder aus Gemischen von Schaf-, Ziegen- oder Büffelmilch" Referenzmethode. Amtliche Sammlung von Untersuchungsverfahren nach § 35 LMBG, Methode 03.52-1 (EG), September 1997, Herausgeber und Redaktion: bgw, Bundesinstitut für gesundheitlichen Verbraucherschutz und Veterinärmedizin, Band I/1b, Lebensmittel (L), Teil 1 b, Beuth Verlag GmbH, Berlin, Köln, Wien, Zürich) erfolgten. Für die Anwendung dieser Methoden sind relativ große Ausgangsmengen löslicher Proteine erforderlich.

Immunoenzymatische Nachweise können ebenfalls zur Tierartbestimmung eingesetzt werden ("Immunoenzymatischer Nachweis der Tierart bei erhitztem Fleisch- und Fleischerzeugnissen; ELISA-Verfahren im Mikrotitertestsystem", Amtliche Sammlung von Untersuchungsverfahren nach § 35 LMBG, Methode 06.00-47, November 1999, Herausgeber und Redaktion: bgvv, Bundesinstitut für gesundheitlichen Verbraucherschutz und Veterinärmedizin, Band I/1c, Lebensmittel (L), Teil 1 c, Beuth Verlag GmbH, Berlin, Köln, Wien, Zürich). Hierbei schwankt die Sensitivität bei unterschiedlichen Tierarten erheblich.

Eine nicht proteinbezogene Methode ist die Kapillargaschromatographie, die zur Auftrennung derivatisierter Fettsäuren eingesetzt wird (Nachweis von rohem und erhitztem Rind- und Schweinefleisch in Fleisch und Fleischerzeugnissen, Screening-Verfahren, Amtliche Sammlung von Untersuchungsverfahren nach § 35 LMBG, Methode 60.00.12, Februar 1996, Herausgeber und Redaktion: bgvv, Bundesinstitut für gesundheitlichen Verbraucherschutz und Veterinärmedizin, Band 1/3, Lebensmittel (L), Teil 2, Beuth Verlag GmbH, Berlin, Köln, Wien, Zürich). Diese Methode ist auf Proben, die keine Fettsäuren enthalten, nicht anwendbar.

Ebenfalls angewendet werden nukleinsäurebasierende Methoden, wobei insbesondere die Anwendung der Polymerasekettenreaktion (PCR) zu nennen ist, bei der entweder direkt artspezifische Nukleinsäuresequenzen nachgewiesen werden oder aber ubiquitäre Sequenzen amplifiziert und danach durch Restriktionsverdau auf artspezifische Sequenzunterschiede untersucht werden. Diese Methoden sind jedoch essentiell an das Vorhandensein amplifizierbarer Nukleinsäuren gebunden ("Genetisches Analyseverfahren zur Abstammungsüberprüfung biologischer Materialien durch Verwendung artspezifischer Primer", DE 198 42 991 A1).

Aus der DE 197 13 194 A1 sind ein Verfahren und eine Anordnung zum Erkennen komplexer Gas-, Geruchs-, Aromamuster einer jeweiligen Substanz auf der Basis der Massenspektroskopie bekannt, das zeitsparende vergleichende massenspektrometrische Bewertungen von der Referenz zugeordneten Serienproben, z.B. von Lebensmitteln, ermöglicht.

Aus SU-A-1 222 246 ist ein Verfahren zur Bestimmung der Art eines Tiers auf der Grundlage von Haaren bekannt, wobei eine Haarprobe einer Totalhydrolyse unterworfen wird, um die daraus resultierenden Aminosäuren zu analysieren. Die Bestimmung von Bakterien unter Verwendung des MALDI-TOFMS-Verfahrens, das eine Analyse von Komplexmischungen von Proteinen bzw. Peptiden erlaubt, ist der Druckschrift RAPID COMMUNICATIONS IN MASS SPECTROMETRY, Bd. 10, Nr. 8, Juni 1996 (1996-06), Seiten 883-888, XP009013355, ISSN 0951-4198 zu entnehmen.

Aufgabe der Erfindung ist es somit, ein Verfahren zur qualitativen und/oder quantitativen Bestimmung von Gattung, Art, Rasse und/oder geographischer Herkunft biologischer Materialien zu schaffen, mit dem mit vertretbarem Aufwand eine Speziesidentifizierung und auch eine quantitative Analyse von Mischungen biologischer Materialien verschiedener Spezies erfolgen kann.

Diese Aufgabe wird erfindungsgemäß durch den Gegenstand der Ansprüche 1 bis 11 gelöst.

Die Vorteile des erfindungsgemäßen Verfahrens bestehen im wesentlichen darin, daß ein Verfahren geschaffen wird,
1. das vollständig unabhängig von morphologischen Merkmalen ist,
2. das bereits mit kleinsten Probenmengen (z.B. ab einer Probenmenge von 20 µg) sichere Aussagen ermöglicht,
3. das die Verwendung von Proben ermöglicht, welche nahezu unlösliche Proteine enthalten,
4. wobei keine immunologischen Wechselwirkungen benötigt werden,
5. wobei die Verwendung von Proben möglich ist, die frei von Fettsäuren sind,
6. das die Verwendung von Proben ermöglicht, die frei von Nukleinsäuren sind,
7. und mit dem hohe Durchsatzraten (mehrere hundert Proben pro Tag) möglich sind.

Die Verwendung des erfindungsgemäßen Verfahrens ist Gegenstand des Patentanspruchs 12.

Bei der erfindungsgemäßen Vorgehensweise werden kurz zusammengefaßt fibrilläre Strukturen aus Federn, Daunen, Schuppen, Haaren oder Horn ohne vorherige Löslichmachung von Struktur-Proteinen direkt enzymatisch-spezifisch zu einem Pool von Spaltpeptiden gespalten. Es erfolgt dabei weder eine Totalhydrolyse zu Aminosäuren, noch wird ein spezieller Analyt gesucht. Der erhaltene Pool von Spaltpeptiden aus fibrillären Struktur-Proteinen wird ohne weitere Trenn-oder Isoliertechniken bevorzugt der MALDI-ToF-Massenspektroskopie unterworfen und die erhaltenen Massenspektrogramme mit Referenz-Massenspektrogrammen abgeglichen. Geeignete spezifische Massenpeaks werden zur Quantifizierung von artfremden Beimengungen von Federn, Daunen, Schuppen, Horn und/oder Haaren herangezogen.

Im Gegensatz zu Probenvorbereitungen von elektrophoretischen oder elektrofokussienden Methoden, die eine Solubilisierung schwerlöslicher Proteine mit anschließender elektrophoretischer bzw. elektrofokussierender Trennung zum Ziel haben, werden bei der vorliegenden Erfindung keine Techniken verwendet, die zu einer Solubilisierung von Proteinen zu einem Protein-Pool mit nachfolgender Trennung führen. Weiterhin erfolgt weder eine Anreicherung bzw. Isolierung spezifischer Proteine, noch erfolgen Spaltungen von einzelnen isolierten Proteinen zwecks Aminosäuresequenzanalysen mit anschließendem Vergleich mit Referenzsequenzen. Es erfolgt bei der erfindungsgemäßen Vorgehensweise kein Vergleich von Proteinbanden-Mustern mit Referenzmustern.

Bei der erfindungsgemäßen Vorgehensweise erfolgt auch im Gegensatz zu Methoden der Spurenanalyse, die auf der Suche nach spezifischen Anlalyten, meist Rückständen anorganischer Gifte (z.B. Arsen) oder organischer Drogen (z.B. Kokain), die die gesuchten Analyten umgebende Protein-Matrix der Haare auflösen, weder eine unspezifische Hydrolyse noch eine Totalhydrolyse der vorliegenden Keratinstrulcturen, noch wird ein spezifischer Analyt gesucht.

Im Gegensatz zu Darstellungen unspezifischer Extrakte aus biologischen Matrices, wie in der DE 197 13 194 A1, mit anschließender Chromatographie und Vergleich der Kurvenverläufe mit Referenzkurven handelt es sich bei der vorliegenden Erfindung um spezifische, enzymatische Spaltungen fibrillärer Strukturen mit definierten Spaltstellen. Die Größe, Anzahl und Sequenzen der Spaltstücke unterliegen dabei direkt den primären Aminosäuresequenzen der fibrillären Proteine, aus denen sie hervorgegangen waren. Die Aminosäuresequenzen sind jedoch genetisch determiniert und somit artspezifisch, sodaß auch zumindest ein Teil der Spaltpeptide artspezifisch sind. Dies unterscheidet die erfindungsgemäße Vorgehensweise von Darstellungen unspezifischer Extrakte aus biologischen Matrices, die je nach Lage des Sekundärstoffwechsels, des Alters, der Umgebung, des Klimas, etc. höchst unterschiedlich sein können.

Die erfindungsgemäße Vorgehensweise wird nachfolgend ausführlich auch anhand von Beispielen beschrieben.

Die biologischen Materialien werden in einem ersten Schritt einer Behandlung unterzogen, während der vorhandene Disulfidbrücken vorteilhaft chemisch behandelt, bevorzugt durch Oxidation oder Reduktion, besonders bevorzugt durch Reduktion, und ganz besonders bevorzugt durch β- Mercaptoethanol reduktiv gespalten werden. Daran schließt sich eine spezifische Spaltung an, wobei die Proben vorteilhaft chemisch oder enzymatisch behandelt werden, bevorzugt enzymatisch behandelt werden, besonders bevorzugt durch hydrolytisch spaltende Enzyme behandelt werden und ganz besonders bevorzugt durch Trypsin, Chymotrypsin, Endoproteinase Glu-C (V8-Protease), Endoproteinase Lys-C, Endoproteinase Arg-C, Endoproteinase Asp-N, Thrombin, Papain, Pepsin, Plasmin oder Mischungen solcher Enzyme behandelt werden.

Die entstehenden Spaltprodukte werden analysiert, vorteilhaft durch HPLC ( high performance liquid chromatography kapillarelektrophoretische Methoden und massenspezifische Detektionsmethoden, bevorzugt durch massenspezifische Detektionsmethoden, besonders bevorzugt durch APCI (atmospheric pressure chemical ionization), CI (chemical ionization), EI (electron ionization), ESI (electrospray ionization), FAB (fast atom bombardment), FD (field desorption), FI (field ionization), LILBID (laser induced liquid beam ionization desorption), LSIMS (liquid secondary ion mass spectrometry), MALDI (matrix assisted laser desorption ionization), PB (particle beam), PD (plasma desorption), SIMS (secondary ion mass spectrometry) oder TSP (thermospray), ganz besonders bevorzugt durch MALDI-TOF MS (matrix assisted laser desorption ionizationtime-of-flight-mass-spectrometry).

Nach der Messung werden die Spektrogramme und Peaktabellen ausgedruckt. Zur Unterscheidung der einzelnen Daten werden die Massenpeaks verwendet, die jeweils nur bei einer der beiden Arten auftreten, in allen untersuchten Proben einer Art nachzuweisen sind und deren Massenisotope nicht die Massenisotope anderer Bruchstücke überlagern. Der Abstand des Jeweils größten Massepeaks ist dabei bevorzugt > 5 Da, besonders bevorzugt > 8 Da. Eine Probe gilt als sicher zugeordnet, wenn sie sich in mindestens drei spezifischen Peaks von der anderen Art unterscheidet.

Als Referenzmaterial wurden handverlesene Daunen verwendet, die von dazu eingesetztem Fachpersonal der Fa. Brinkhaus, D-48231 Warendorf zur Verfügung gestellt wurden.

### Beispiel 1

### Erstellung von Referenzdaten anhand von je zehn sicher klassifizierten Daunen von Enten und Gänsen

Je 100 µl einer Lösung aus 25 mM NH4HC03 (Merck Darmstadt) mit 5 Vol. % ßMercaptoethanol (Merck, Darmstadt) wurden in zwanzig 1,5 ml Eppendorf Safelock-Reaktionsgefäße pipettiert. Pro Gefäß wurde eine Daune von Ente oder Gans unter Zuhilfenahme einer schlanken, glatten Pinzette überführt, wobei darauf geachtet wurde, daß die Daunen von der Flüssigkeit gut benetzt wurden.

Die Gefäße wurden verschlossen und in einem geeigneten Halter für zwanzig Minuten in einem kochenden Wasserbad inkubiert. Danach wurden die Gefäße dem Wasserbad entnommen und auf Eis abgekühlt. Pro Gefäß werden 100 µl einer Lösung aus 25 MM NH4HC03 und 5 mg/ml Trypsin (spezifisch Aktivität 1645 U/mg, Merck Darmstadt) zupipettiert. Es folgte eine zweistündige Inkubation im Wasserbad bei 37° C. Aus jedem Ansatz wurden 10 µl entnommen und mit jeweils 90 µl einer Lösung aus saturierter α-Cyano4-hydroxyzimtsäure (Sigma, München) in 30 % Acetonitril (Merck, Darmstadt), 1 % Trifluoressigsäure (Fluka, Seelze) gemischt (vortexen). 1 µl der Lösung wurde auf die MALDI-ToF-Targetplatte aufgetragen und bei Raumtemperatur verdampft (Abtrocknung).

Die entstandenen Hydrolyseprodukte wurden mit einem MALDI-ToF-Massenspektrometer, Typ Reflex III, der Fa. Bruker, Bremen, manuell gemessen.

Ein gepulster Stickstofflaser der Wellenlänge von X= 337 nm mit der Pulsdauer von 3 ns wurde zur Desorption und lonsisation von Matrix-Proben-Cokristallen eingesetzt. Im Massenbereich von 1000 bis 2200 Da wurde mit gepulster Ionenextraktion gemessen und positiv geladene Ionen im Reflektron-Modus detektiert. Die angelegten Spannungen betrugen 20 KV an der Targetplatte und 20 KV bzw. 16,4 KV an der ersten Extraktionsplatte. Die Grundplatte war spannungslos, während die Linsenspannungen 9,6 KV betrugen und eine Reflektronspannung von 23 KV anlag. 100 Spektren mit einer Laserabschwächung von 75-60 % wurden aufsummiert und die Massen der detektierten Spaltprodukte mithilfe von Massen-Kalibrierstandards (ACTH- Clip (18- 39, human), Angiotensin 2, Somatostatin und Substanz P (alle Sigma, München)) bestimmt.

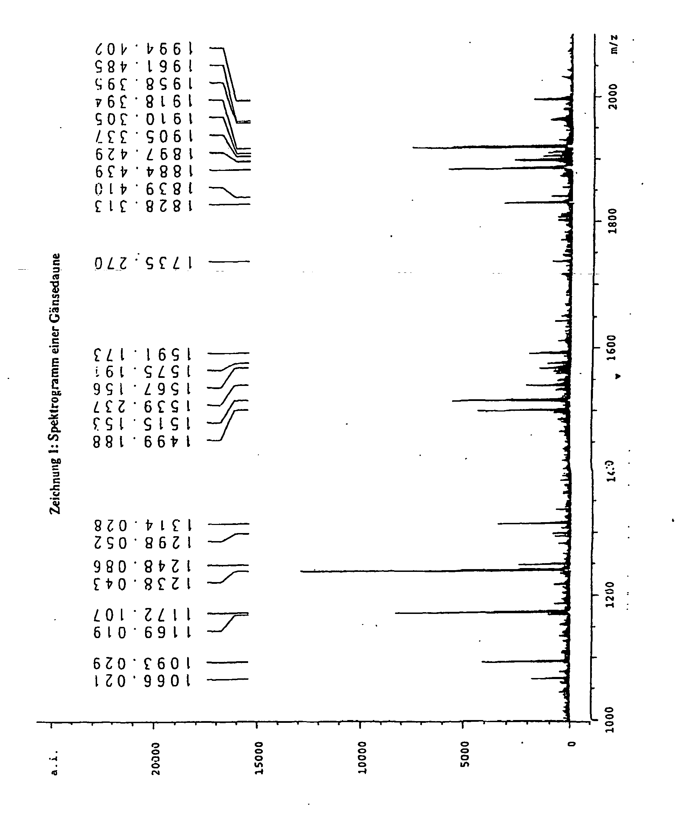

Zeichnung 1 zeigt ein Massenspektrogramm einer Gänsedaune mit allen erfaßten Massepeaks im Bereich von 1000-2200 Da.

In Tabelle 1 sind alle gemessenen Massepeaks im Bereich von 1000-2200 Da aufgelistet, deren relative Intensität größer als 2 % des größten Massepeaks ist.

**Tabelle 1: Peakreport zu Zeichnung 1 (Gans)**

| ###4############# PEAK LISTING #################### | | | |
|---|---|---|---|
| # ADDRESS # | MASS [m/z] | RELATIVE INTENSITY | artspezifische Peaks für Gans |
| 1 | 1958.3949 | 0.0221 | |
| 2 | 1298.0518 | 0.0713 | |
| 3 | 1735.2703 | 0.0597 | |
| 4 | **1905.3366** | 0.0691 | spezifisch Gans |
| 5 | 1839.4101 | 0.0681 | |
| 6 | 1961.4852 | 0.0821 | |
| 7 | 1169.0187 | 0.0903 | |
| 8 | 1575.1906 | 0.0954 | |
| 9 | 1910.3051 | 0.0826 | |
| 10 | 2576.9205 | 0.0710 | |
| 11 | 1567.1564 | 0.1086 | |
| 12 | 1994.4024 | 0.1244 | |
| 13 | 1066.0209 | 0.1391 | |
| 14 | 1591.1734 | 0.1532 | |
| 15 | 1539.2371 | 0.1543 | |
| 16 | 3514.4661 | 0.0752 | |
| 17 | 2211.7209 | 0.1341 | |
| 18 | 1248.0860 | 0.1787 | |
| 19 | 1897.4291 | 0.1881 | |
| 20 | **1828.3129** | 0.2418 | spezifisch Gans |
| 21 | 3726.9439 | 0.0629 | |
| 22 | **1314.0278** | 0.2606 | spezifisch Gans |
| 23 | 2283.8084 | 0.2170 | |
| 24 | 1093.0285 | 0.3319 | |
| 25 | 1499.1882 | 0.3000 | |
| 26 | 1515.1526 | 0.4301 | |
| 27 | **1884.4389** | 0.4095 | spezifisch Gans |
| 28 | 1918.3936 | 0.5399 | |
| 29 | 1172.1066 | 0.6766 | |
| 30 | **1238.0426** | 0.9958 | spezifisch Gans |

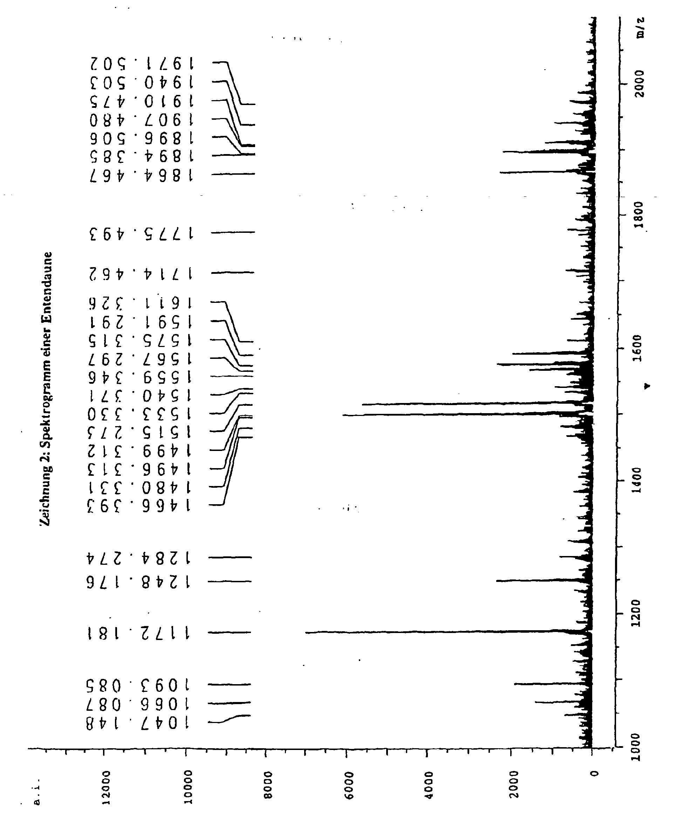

Zeichnung 2 zeigt ein Massenspektrogramm einer Entendaune mit allen erfaßten Massepeaks im Bereich von 1000-2200 Da. In Tabelle 2 sind alle gemessenen Massepeaks im Bereich von 1000-2200 Da aufgelistet, deren relative Intensität größer als 7 % des größten Massepeaks ist.

**Tabelle 2: Peakreport zu Zeichnung 2 (Ente)**

| *#################* PEAK LISTING ##################### | | | |
|---|---|---|---|
| # ADDRESS | MASS | RELATIVE | artspezifische |
| # | [m/z] | INTENSITY | Peaks für Ente |
| 1 | **1971.5021** | 0.0795 | spezifisch Ente |
| 2 | 1907.4800 | 0.0551 | |
| 3 | 1611.3260 | 0.0821 | |
| 4 | 1466.3930 | 0.0934 | |
| 5 | 1559.3456 | 0.1070 | |
| 6 | 1775.4928 | 0.0688 | |
| 7 | 1047.1479 | 0.0886 | |
| 8 | 1533.3302 | 0.0969 | |
| 9 | 1714.4620 | 0.0891 | |
| 10 | 1480.3308 | 0.0950 | |
| 11 | 1284.2740 | 0.1101 | |
| 12 | 1496.3128 | 0.1029 | |
| 13 | 1540.3711 | 0.1219 | |
| 14 | 1940.5031 | 0.1170 | |
| 15 | 1910.4754 | 0.1460 | |
| 16 | 3727.1595 | 0.0392 | |
| 17 | 1066.0866 | 0.1912 | |
| 18 | 2211.8373 | 0.1639 | |
| 19 | 1567.2971 | 0.2097 | |
| 20 | **1894.3853** | 0.1099 | spezifisch Ente |
| 21 | 1093.0849 | 0.2828 | |
| 22 | 1591.2911 | 0.2834 | |
| 23 | 1896.5056 | 0.2689 | |
| 24 | 1864.4674 | 0.2784 | |
| 25 | 1248.1755 | 0.3154 | |
| 26 | 1575.3149 | 0.3464 | |
| 27 | 2283.9558 | 0.2817 | |
| 28 | 1515.2727 | 0.7978 | |
| 29 | 1499.3124 | 0.8917 | |
| 30 | 1172.1808 | 1.0140 | |

In Tabelle 1 und 2 sind diejenigen Massepeaks markiert, die für die Identifizierung von Gänsedaunen in einem Gemisch von Gänse- und Entendaunen geeignet sind.

Zur Unterscheidung der einzelnen Daten wurden nur die Massenpeaks verwendet, die jeweils nur bei einer der beiden Arten auftreten und in allen untersuchten Proben einer Art nachzuweisen waren. Eine Probe gilt als sicher zugeordnet, wenn sie sich in mindestens drei ,spezifischen Peaks von der anderen Art unterscheidet.

### Beispiel 2

### Analyse eines unbekannten Probengemisches aus Daunen

Aus einem unbekannten, möglichst homogenen Probengemisch wurden mit Hilfe einer Pinzette Probencluster entnommen und mittels einer Feinwaage (Sartorius, Göttingen, Typ BP 221 S) so lange zugewogen, bis ein Probengewicht von mindestens 110 mg erreicht ist. Die Entnahme erfolgte zufällig, ohne daß Größe, Form oder Färbung des Probenmaterials beachtet wurden. Aus der so erhaltenen Stichprobe wurden nun die Daunen sowie die vorhandenen Bruchstücke mit Hilfe einer spitzen Pinzette entnommen und einzeln auf einer Ultrafeinwaage (Sartorius, Typ SC 2, Wägebereich bis 0,1 µg gewogen. Die Gewichte wurden probenbezogen notiert, die isolierten Strukturen wurden getrennt in durchnummerierte 0,2 ml PCR-Gefäße (Achtersbrips) überführt, in die jeweils 50 µl einer Lösung aus 25 mM NH4HCO3 mit 5 Vol. % β-Mercaptoethanol vorpipettiert waren. Dabei wurde auf eine ausreichende Benetzung der Proben geachtet. Die Strips wurden verschlossen und in einen auf 99,9° C vorgeheizten PCR- Cycler (Biometra, Göttingen, Uno- Thermoblock, 96 wells) eingestellt (Heizdeckel vorgeheizt auf 108° C). Der Cycler wurde so programmiert, daß die Temperatur nach 20 Minuten auf 4° C absinkt (Haltephase mit einer Achtkanalpipette werden pro Cap 50 µl 25 mM NH4HCO3 mit 5 mg/ml Trypsin spez. Aktivität 1645 U/mg) zupipettiert. Nach erneutem Verschließen der Strips wurde der Cycler auf 37° C aufgeheizt und kühlte nach 2 h automatisch auf 4° C (Haltephase). Nach Ablauf der Reaktion wurden mit der Achtkanalpipette je 5 µl des Reaktionsgemisches entnommen und in die Gefäße eines weiteren Strips überführt, in welchen 45 µl saturierter (α-Cyano-4-hydroxyzimtsäure in 30 % Acetonitril, 1 % Trifluoressigsäure pro Cap vorpipettiert waren. Die Proben wurden durch Pipettieren gemischt. Danach wurden sie mit der gleichen Pipette direkt auf die Trägerplatte aufgetragen.

Ein gepulster Sticksofflaser der Wellenlänge von X= 337 nm mit der Pulsdauer von 3 ns wurde zur Desorption und Ionsisation von Matrix-Proben-Cokristallen eingesetzt. Im Massenbereich von 1000 bis 2200 Da wurde mit gepulster Ionenextraktion gemessen und positiv geladene Ionen im Reflektron-Modus detektiert. Die angelegten Spannungen betrugen 20 KV an der Targetplatte und 20 KV bzw. 16,4 KV an der ersten Extraktionsplatte. Die Grundplatte war spannungslos, während die Linsenspannungen 9,6 KV betrugen und eine Reflektron-spannung von 23 KV anlag. Je 100 Spektren mit besserem Signal-Rauschverhältnis als 4, einem Noiserange besser als 100 und einer Peakauflösung von besser als 1400 wurden automatisch aufsummiert und die Massen der detektierten Spaltprodukte mithilfe von Massen-Kalibrierstandards bestimmt.

Die Messung der hundert Proben erfolgte automatisch im Autoexecute- Modus.

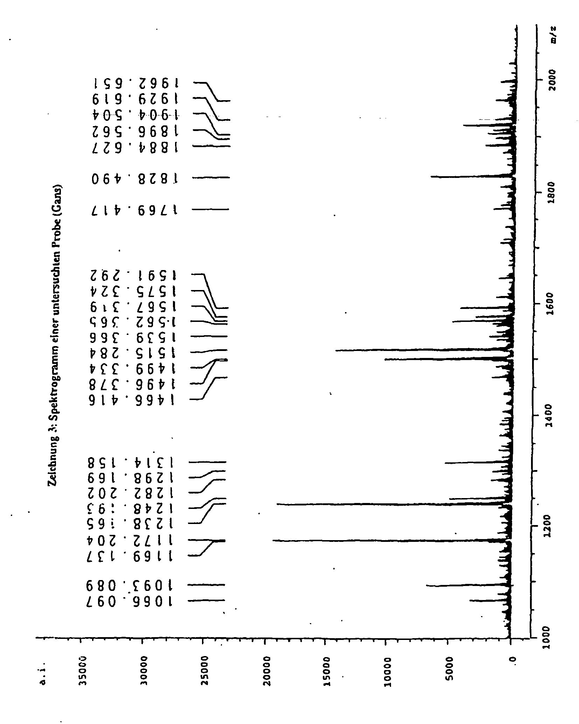

Zeichnung 3 zeigt ein Massenspektrogramm einer zunächst unbekannnten Daune. In Tabelle 3 sind die zugehörigen Massepeaks mit >6 % der relativen Intensität des höchsten Massepeaks im Bereich von 1000-2200 Da aufgelistet. In dieser Tabelle sind die als charakteristisch identifizierten Peaks fett gedruckt. Die Identifikation dieser Peaks ist im Beispiel 1 beschrieben.

**Tabelle 3: Peakreport zu Zeichnung 3 unbekannte Probe**

| ################# PEAK LISTING ##################### | | |
|---|---|---|
| # | ADDRESS | MASSRELATIVE |
| # | | [m/z]INTENSITY |
| 1 | 2155.8426 | 0.0623 |
| 2 | 1298.1691 | 0.0860 |
| 3 | 1962.6507 | 0.0760 |
| 4 | 1896.5622 | 0.0776 |
| 5 | 1282.2015 | 0.0961 |
| 6 | 1929.6189 | 0.0808 |
| | 17169.14168 | 0.0865 |
| 8 | 1562.3649 | 0.0950 |
| 9 | 1466.4159 | 0.0983 |
| 10 | 1496.3776 | 0.0821 |
| 11 | 1169.1366 | 0.1027 |
| 12 | 1539.3662 | 0.1049 |
| 13 | **1904.5043** | 0.1050 |
| 14 | **1884.6271** | 0.1144 |
| 15 | 3726.9230 | 0.0583 |
| 16 | 1575.3237 | 0.1597 |
| 17 | 1066.0971 | 0.1574 |
| 18 | 1591.2919 | 0.2131 |
| 19 | 1567.3186 | 0.2445 |
| 20 | 1248.1932 | 0.2535 |
| 21 | **1314.1583** | 0.2711 |
| 22 | **1828.4897** | 0.3321 |
| 23 | 1093.0894 | 0.3357 |
| 24 | 2211.8909 | 0.2932 |
| 25 | 2284.0054 | 0.4047 |
| 26 | 1499.3345 | 0.5357 |
| 27 | 1515.2838 | 0.7357 |
| 28 | **1238.1652** | 1.0721 |
| 29 | 1172.2036 | 1.0207 |

In Tabelle 4 sind die detektierten, charakteristischen Peaks einer bekannten Gänse bzw. Entendaune dargestellt. In der unbekannten Probe wurden alle Massepeaks, die für Gänse charakteristisch sind, gefunden, während keine Enten- spezifischen Massepeaks detektiert wurden. Die unbekannte Daune wurde aufgrund der in Tabelle 4 detektierten, bzw. nicht detektierten Massepeaks eindeutig als Gänsedaune identifiziert.

**Tabelle 4: Zuordnungstabelle Ente / Gans**

| Peakmasse (m/z) | Gans- spezifisch | Enten- spezifisch | untersuchte Probe |
|---|---|---|---|
| 1238,043 | + | - | + |
| 1314,028 | + | - | + |
| 1828,313 | + | - | + |
| 1884,439 | + | - | + |
| 1894,385 | - | + | - |
| 1905,337 | + | - | + |
| 1971,502 | - | + | - |

## Patentansprüche

1. Verfahren zur qualitativen und/oder quantitativen Bestimmung von Gattung, Art, Rasse und/oder geographischer Herkunft biologischer Materialien auf der Grundlage von Schuppen, Haaren, Federn, Daunen und/oder Horn, **dadurch gekennzeichnet, daß**
a) die Schuppen, Haare, Federn, Daunen und/oder das Horn oder Teile davon durch spezifische chemische oder biokatalytische Umsetzungen in einen Pool von Spaltpeptiden oder Derivate dieser Spaltpeptide umgesetzt werden,
b) diese Spaltpeptide oder Derivate dieser Spaltpeptide massenspektrometrisch einzeln oder in Gruppen detektiert werden,
c) über einzelne Analysensignale oder Gruppen von Signalen durch Vergleich mit Referenzproben qualitativ und/oder quantitativ Gattung, Art, Rasse und/oder geographische Herkunft bestimmt wird.

2. Verfahren nach Anspruch 1, **dadurch gekennzeichnet, daß** in Schritt a) Disulfidbrücken aufspaltende reduzierende oder oxidierende Reagenzien zugegeben werden.

3. Verfahren nach Anspruch 1 oder 2, **dadurch gekennzeichnet, daß** in Schritt a) Schwefelbrücken reduzierende oder oxidierende Reagenzien eingesetzt werden, die eine oder mehrere funktionelle Gruppen aus den Substanzklassen der Thiole Sulfide, Sulfoxide, Sulfone, Sulfonamide, Peroxide, Metallkatalysatoren, Phosphine, Phosphite, Phosphate, Halogenide, Oxirane, Alkine, Olefine, Amide, Amine, Carbonsäuren, Carbonsäureester, Alkohole, Aldehyde oder Ketone umfassen.

4. Verfahren nach Anspruch 1 oder 2, **dadurch gekennzeichnet, daß** in Schritt a) chemische Hydrolysereagenzien für Biopolymere eingesetzt werden

5. Verfahren nach Anspruch 1 oder 2, **dadurch gekennzeichnet, daß** die Umsetzung in Schritt a) mit hydrolytisch spaltenden Enzymen, insbesondere mit Trypsin, Chymotrypsin, Endoproteinase Glu-C (V8-Protease), Endoproteinase Lys-C, Endoproteinase Arg-C, Endoproteinase Asp-N, Thrombin, Papain, Pepsin, Plasmin oder Mischungen solcher Enzyme, durchgeführt wird.

6. Verfahren nach Anspruch 5, **dadurch gekennzeichnet, daß** als Biokatalysatoren Bakterien, Pilze, pflanzliche, tierische oder humane Zellen oder Gewebe oder Kombinationen dieser bzw. Enzyme, Antikörper, Proteine Riboenzyme, Peptide oder sonstige Biokatalysatoren sowie Kombinationen dieser eingesetzt werden.

7. Verfahren nach den Ansprüchen 2-6, **dadurch gekennzeichnet, daß** als spezifisches Detektionssystem der gebildeten Bruchstücke eine massenspektrometrische Ionisierungsmethode, insbesondere APCI (atmospheric pressure chemical ionization), CI (chemical ionization), EI (election ionization), ESI (electrospray ionization), FAB (fast atom bombardment), FD (field desorption), FI (field ionization), LILBID (laser induced liquid beam ionization desorption), LSIMS (liquid secondary ion mass spectrometry), MALDI (matrix assisted laser desorption ionization), PB (particle beam), PD (plasma desorption), SIMS (secondary Ion mass spectrometry), oder TSP (thermospray) oder eine Kombination solcher Ionisierungsmethoden, eingesetzt wird.

8. Verfahren nach den Ansprüchen 2-6, **dadurch gekennzeichnet, daß** die gebildeten Bruchstücke mit Flüssigkeitschromatographie, insbesondere LC (liquid chromatography), MPLC (middle pressure liquid chromatography), HPLC. (high performance liquid chromatography), aufgetrennt und detektiert werden.

9. Verfahren nach den Ansprüchen 2-8, **dadurch gekennzeichnet, daß** die gebildeten Bruchstücke mittels kapillarelektrophoretischer Methoden aufgetrennt und danach detektiert werden.

10. Verfahren nach den Ansprüchen 2-9, **dadurch gekennzeichnet, daß** die Bearbeitung einer Serie von Proben mit Hilfe eines Roboters und/oder unter Verwendung von Misch-Heiz- und Kühlgeräten durchgeführt wird.

11. Verfahren nach Anspruch 10, **dadurch gekennzeichnet, daß** die Proben von einem oder mehreren Robotern aus einer oder mehreren Mikrotiterplatten in ein oder mehrere Analysengeräte transferiert werden.

12. Verwendung des Verfahrens nach den Ansprüchen 1-11 zur Identifizierung der Herkunft biologischer Materialien, insbesondere biologischer Materialien, die strukturbildende Proteine oder deren Derivate enthalten.

## Claims

1. A method for the qualitative and/or quantitative determination of genus, species, breed and/or geographical origin of biological materials on the basis of scales, hair, feathers, down and/or horn, **characterised in that**
a) the scales, hair, feathers, down and/or horn, or parts thereof, are converted by means of specific chemical or bio-catalytic conversions into a pool of cleavage peptides or derivatives of these cleavage peptides,
b) these cleavage peptides or derivatives of these cleavage peptides are detected individually or in groups by means of mass spectrometry,
c) genus, species, breed and/or geographic origin of the material is determined qualitatively and/or quantitatively using individual analysis signals or groups of signals and comparing them with those of reference samples.

2. The method according to claim 1, **characterised in that** in step a), reagents are added that cleave, reduce or oxidise disulfide bonds.

3. The method according to claim 1 or 2, **characterised in that** in step a), reagents are used that reduce or oxidise sulfur-sulfur bonds and that contain one or more functional groups selected from the substance classes thiols, sulfides, sulfoxides, sulfones, sulfonamides, peroxides, metal catalysts, phosphines, phosphites, phosphates, halides, oxiranes, alkynes, olefins, amides, amines, carboxylic acids, carboxylates, alcohols, aldehydes or ketones.

4. The method according to claim 1 or 2, **characterised in that** in step a), chemical hydrolysing reagents for bio-polymers are used.

5. The method according to claim 1 or 2, **characterised in that** the conversion in step
a) is performed with hydrolytic cleaving enzymes, especially with trypsin, chymotrypsin, endoproteinase Glu-C (V8 protease), endoproteinase Lys-C, endoproteinase Arg-C, endoproteinase Asp-N, thrombin, papain, pepsin, plasmin or mixtures of such enzymes.

6. The method according to claim 5, **characterised in that** bacteria, fungi, plant cells, animal cells, human cells or tissue or combinations thereof, enzymes, antibodies, proteins, ribo-enzymes, peptides or other biological catalysts as well as combinations thereof are used as biological catalysts.

7. The method according to the claims 2 to 6, **characterised in that** a mass-spectrometric ionisation method, especially atmospheric pressure chemical ionisation (APCI), chemical ionisation (Cl), election ionisation (El), electrospray ionisation (ESI), fast atom bombardment (FAB), field desorption (FD), field ionisation (Fl), laser induced liquid beam ionisation desorption (LILBID), liquid secondary ion mass spectrometry (LSIMS), matrix assisted laser desorption ionisation (MALDI), particle beam (PB), plasma desorption (PD), secondary ion mass spectrometry (SIMS), thermospray (TSP) or a combination of such ionisation methods is used as a specific detection system to detect the fragments generated.

8. The method according to the claims 2 to 6, **characterised in that** the fragments generated are separated and detected by liquid chromatography, especially liquid chromatography (LC), medium pressure liquid chromatography (MPLC) and high performance liquid chromatography (HPLC)..

9. The method according to the claims 2-8, **characterised in that** capillary electrophoretic methods are used to separate and subsequently detect the fragments generated.

10. The method according to the claims 2-9, **characterised in that** a series of samples is processed by means of a robot and/or through use of mixing, heating and cooling equipment.

11. The method according to claim 10, **characterised in that** the samples are transferred from one or more microtiter plates to one or more analytical devices by one or more robots.

12. Use of the method according to the claims 1-11 for identifying the origin of biological materials, especially of biological materials which contain structure-forming proteins or their derivatives.

## Revendications

1. Procédé pour la détermination qualitative et/ou quantitative du genre, de l'ordre, de la race et/ou de l'origine géographique de matières biologiques sur la base d'écailles, de poils, de plumes, de duvet et/ou de corne, **caractérisé en ce que**
a) les écailles, les poils, les plumes, les duvets et/ou la corne ou des parties de ceux-ci sont décomposés par des décompositions chimiques ou biocatalytiques spécifiques dans un pool de fragments de peptides ou de dérivés de ces fragments de peptides,
b) ces fragments de peptides ou dérivés de fragments de peptides sont détectés séparément ou en groupe par spectrométrie de masse,
c) le genre, l'ordre, la race et/ou l'origine géographique sont déterminés qualitativement et/ou quantitativement sur la base des signaux d'analyse individuels ou de groupes de signaux par comparaison avec des échantillons de référence.

2. Procédé selon la revendication 1, **caractérisé en ce que** dans l'étape a) des réactifs décomposant réduisant ou oxydant les ponts bisulfure sont ajoutés.

3. Procédé selon la revendication 1 ou 2, **caractérisé en ce que** dans l'étape a) des réactifs réduisant ou oxydant les ponts soufre qui comprennent un ou plusieurs groupes fonctionnels parmi les classes de substances des thiols, sulfures, sulfoxydes, sulfones, sulfonamides, péroxydes, catalyseurs métalliques, phosphines, phosphites, phosphates, halogénures, oxyranes, alcynes, oléfines, amides, amines, acides carboxyliques, esters d'acides carboxyliques, alcools, aldéhydes ou cétones.

4. Procédé selon la revendication 1 ou 2, **caractérisé en ce que** dans l'étape a) des réactifs chimiques d'hydrolyse pour des biopolymères sont utilisés.

5. Procédé selon la revendication 1 ou 2, **caractérisé en ce que** la décomposition de l'étape a) est réalisée avec des enzymes de dédoublement hydrolytique, notamment avec de la trypsine, chymotrypsine, de l'endoproteinase Glu-C (protéase V8), de l'endoprotinase Lys-C, de l'endoproteinase Arg-C, de l'endoproteinase Asp-N, de la thrombine, de la papaïne, de la pepsine, de la plasmine ou des mélanges de telles enzymes.

6. Procédé selon la revendication 5, **caractérisé en ce qu'**on utilise comme biocatalyseur des bactéries, des champignons, des cellules ou des tissus végétaux animaux ou humains ou des combinaisons de ceux-ci, ou bien des enzymes, des anticorps, des protéines, ribozymes, des peptides ou d'autres biocatalyseurs ainsi que des combinaisons de ceux-ci.

7. Procédé selon les revendications 2 à 6, **caractérisé en ce qu'**on utilise comme système de détection spécifique des fragments formés une méthode d'ionisation par spectrométrie de masse, notamment l'APCI (atmospheric pressure chemical ionization), la Cl (chemical ionization), l'El (electron ionization), l'ESI (electroyspray ionization), le FAB (fast atom bombardment), la FD (field desorption), la FI (field ionization), la LILBID (laser induced liquid beam ionization desorption), la LSIMS (liquid secondary ion mass spectrometry), la MALDI (matrix assisted laser desorption ionization), le PB (particle beam), la PD (plasma desorption), la SIMS (secondary ion mass spectrometry) ou le TSP (thermospray), ou une combinaison de telles méthodes d'ionisation.

8. Procédé selon les revendications 2 à 6, **caractérisé en ce que** les fragments formés sont séparés et détectés par chromatographie liquide, notamment par LC (liquid chromatography), MPLC (middle pressure liquid chromatography), HPLC (high performance liquid chromatography).

9. Procédé selon les revendications 2 à 8, **caractérisé en ce que** les fragments formés sont séparés par électrophorèse capillaire, puis ensuite détectés.

10. Procédé selon les revendications 2 à 9, **caractérisé en ce que** le traitement d'une série d'échantillons est réalisé par un robot et/ou en utilisant des appareils de mélange, de chauffage et de refroidissement.

11. Procédé selon la revendication 10, **caractérisé en ce que** les échantillons sont transférés par un ou plusieurs robots d'une ou plusieurs plaques de microtitration dans un ou plusieurs appareils d'analyse.

12. Utilisation du procédé selon les revendications 1 à 11 pour l'identification de l'origine de matières biologiques, notamment de matières biologiques qui contiennent des protéines formant des structures ou leurs dérivés.
